# EUROPÄISCHE PATENTANMELDUNG

(11) **EP 4 775 260 A2**
(43) Veröffentlichungstag der Anmeldung: **15.07.2026**
(21) Anmeldenummer: 26178949.9
(22) Anmeldetag: 11.06.2019
(51) Int. Cl.: A61N 5/10

(54) **TEILCHENENERGIE-MESSGERÄT UND VERFAHREN ZUM ERMITTELN EINER STRAHLENENERGIE EINES TEILCHENSTRAHLS**

(62) Teilanmeldung aus: 19730328.2
(71) Anmelder: Bundesrepublik Deutschland, vertr. durch das Bundesministerium f. Wirtschaft und Technologie,, 38116 Braunschweig (DE)
(72) Erfinder: Makowski, Christoph, 38116 Braunschweig (DE)
(74) Vertreter: Gramm, Lins & Partner Patent- und Rechtsanwälte PartGmbB

(57) **Zusammenfassung**

Die Erfindung betrifft ein Teilchenenergie-Messgerät (14) zum Bestimmen der Energie eines Teilchenstrahls (26), mit (a) zumindest zwanzig Kondensatoren (30), die (i) jeweils eine erste Kondensatorplatte (32) und (ii) eine zweite Kondensatorplatte (34) aufweisen und (iii) bezüglich einer Strahleinfallsrichtung (S) hintereinander angeordnet sind, (b) einem Multiplexer (46), der (i) einen Multiplexer-Ausgang (48) und (ii) eine Mehrzahl an Multiplexer-Eingängen (50.n) hat, wobei jeder Multiplexer-Eingang (50) mit genau einem Kondensator (30.n) verbunden ist, und der (iii) eingerichtet ist zum Verbinden jeweils einer ersten Kondensatorplatte (32.n) des jeweiligen Kondensators mit dem Multiplexer-Ausgang (48), (a) einem Plattenladungsmesser (62), der (i) einen Plattenladungsmesser-Eingang (64), der mit dem Multiplexer-Ausgang (48) verbunden (48) ist, und (ii) einen Plattenladungsmesser-Ausgang (65) aufweist und (iii) ausgebildet ist zum Messen einer Ladung (Qn), die über den Multiplexer-Ausgang (48) und den Plattenladungsmesser-Eingang (64) fließt, (c) einer Auswerteschaltung (58), die mit dem Plattenladungsmesser (62) und dem Multiplexer (46) verbunden ist und ausgebildet ist zum automatischen (i) Bewirken eines Umschaltens von einem Multiplexer-Eingang (50.n) auf einen anderen Multiplexer-Eingang (50.n), sodass sukzessive die Kondensatoren (30.n) einzeln entladen werden, und (ii) Erfassen der von jedem Kondensator (30.n) beim Entladen fließenden Ladung (Qn), sodass Ladungs-Daten erhalten werden, aus denen die Teilchenenergie (E) berechenbar ist.

## Beschreibung

Teilchenbeschleuniger werden insbesondere in der Forschung und in der Medizin verwendet, um einen Teilchenstrahl aus Teilchen einer vorgegebenen Energie zu erzeugen. In medizinischen Anwendungen wird der Teilchenstrahl verwendet, um gezielt Gewebe zu zerstören. Um benachbartes Gewebe zu schützen, ist es notwendig, dass die Teilchenenergie mit hoher Genauigkeit bekannt ist. Notwendig ist es zudem, dass auch die Anzahl an Teilchen - oder äquivalent dazu die Gesamt-Ladung des Teilchenstrahls - bekannt ist, um die Dosis berechnen zu können, die dem Gewebe appliziert wird.

Beschleuniger bestehen aus einer Vielzahl an Komponenten. Bereits geringfügige Änderungen der Position der Komponenten relativ zueinander beeinflussen die Teilchenenergie deutlich. Es ist daher notwendig, Beschleuniger zumindest bei Inbetriebnahme, zumeist aber auch in regelmäßigen Zeitabständen, zu justieren. Unter dem Justieren ist nicht nur die rein mechanische Ausrichtung zu verstehen sondern auch die späteren Einstellungen, beispielsweise an meist vorhandenen Fokus- und Umlenk-Elektromagneten, Phasenschieber, RF-Leistungssteller und/oder der Teilchenquelle. Diese beeinflussen allesamt unmittelbar die Energie und Leistung des Strahls und müssen im normalen Betrieb oft bedient werden.

Aufgrund der Vielzahl an Komponenten, deren Positionen relativ zueinander relevant sind, und der Tatsache, dass der Einfluss einer Positionsänderung einer Komponente relativ zu den anderen in der Regel unbekannt ist, muss eine große Anzahl an Testmessungen durchgeführt werden, um eine gute Ausrichtung der einzelnen Komponenten des Teilchenbeschleunigers relativ zueinander zu erreichen.

Da eine große Anzahl an Testmessungen durchgeführt werden muss, ist die Zeitdauer für jede einzelne Testmessung sehr relevant für die Gesamtdauer der Justage. Teilchenenergie-Messgeräte sollten daher sowohl eine geringe Messunsicherheit haben als auch eine geringe Messzeit erlauben.

Aus der US 2018/016 4445 A1 ist ein modularer Detektor zum Vermessen eines Partikelstrahls bekannt. Die einzelnen Module besitzen jeweils eine erste Elektrode und eine zweite Elektrode, zwischen denen eine Ionisationskammer ausgebildet ist. In Strahlrichtung hinter der hinteren Elektrode ist ein Dielektrikum ausgebildet, das den Strahl schwächt. Nachteilig an einem solchen Detektor ist der vergleichsweise hohe apparative Aufwand.

Aus der US 5 308 988 A ist ein Flächendetektor bekannt, mittels dem die Strahlintensität quer zur Strahlausbreitungsrichtung vermessen werden kann. Es ist mit einem solchen Flächendetektor nicht möglich, die Energie des Teilchenstrahls mit geringer Messunsicherheit zu vermessen.

In der US 2008/0283764 A1 ist ein Röntgendetektor beschrieben, der die durch Strahlung ausgelöste Veränderung der elektrischen Leitfähigkeit zum Detektieren ausnutzt. Trifft ein Röntgenquant auf den Detektor, ändert sich die Leitfähigkeit zwischen den beiden Platten des Kondensators und es entsteht ein Strompuls, dessen Stärke gemessen wird. Nachteilig auch an einem solchen Detektor ist der vergleichsweise hohe apparative Aufwand.

Der Erfindung liegt die Aufgabe zugrunde, die Messungen der Teilchenenergie und des Teilchenstrahls zu verbessern.

Die Erfindung löst das Problem durch ein Teilchenenergie-Messgerät zum Bestimmen der Energie eines Teilchenstrahls mit (a) zumindest zwanzig Kondensatoren, die (i) jeweils eine erste Kondensatorplatte und (ii) eine zweite Kondensatorplatte aufweisen und (iii) bezüglich einer Strahleinfallsrichtung hintereinander angeordnet sind, (b) einem Multiplexer, der (i) einen Multiplexer-Ausgang und (ii) eine Mehrzahl an Multiplexer-Eingängen hat, wobei jeder Multiplexer-Eingang zum Verbinden mit genau einem Kondensator ausgebildet ist, und der (iii) eingerichtet ist zum Verbinden jeweils einer der Kondensatorplatten des jeweiligen Kondensators mit dem Multiplexer-Ausgang, (c) einem Summenladungsmesser, der (i) einen Summenladungsmesser-Eingang, der mit den zweiten Kondensatorplatten verbunden ist zum Erfassen einer Summenladung der Ladungen auf allen Kondensatoren, und (ii) einen Summenladungsmesser-Ausgang aufweist, und (d) einer Auswerteschaltung, die mit dem Summenladungsmesser und dem Multiplexer verbunden ist und ausgebildet ist zum automatischen (i) Bewirken eines Umschaltens von einem Multiplexer-Eingang auf einen anderen Multiplexer-Eingang, sodass sukzessive die Kondensatoren einzeln entladen werden, und (ii) Erfassen der von jedem Kondensator beim Entladen abfließenden Ladung, sodass Ladungs-Daten erhalten werden, aus denen die Teilchenenergie berechenbar ist.

Die Erfindung löst das Problem zudem durch ein Verfahren zum Ermitteln einer Strahlenergie eines Teilchenstrahls, mit den Schritten:
(a) Einfallen-Lassen des Teilchenstrahls auf ein Teilchenenergie-Messgerät, das
   (i) zumindest zwanzig Kondensatoren, die
      jeweils eine erste Kondensatorplatte und
      eine zweite Kondensatorplatte aufweisen und
      bezüglich einer Strahleinfallsrichtung S hintereinander angeordnet sind,
   (ii) einen Multiplexer, der
      einen Multiplexer-Ausgang und
      eine Mehrzahl an Multiplexer-Eingängen hat, wobei jeder Multiplexer-Eingang mit genau einem Kondensator verbindbar ist, und der
      eingerichtet ist zum Verbinden jeweils einer der Kondensatorplatten des jeweiligen Kondensators mit dem Multiplexer-Ausgang,
   (iii) einen Summenladungsmesser, der
      einen Summenladungsmesser-Eingang, der mit den zweiten Kondensatorplatten verbunden ist, und
      einen Summenladungsmesser-Ausgang aufweist,
(b) sukzessives Messen der Ladungen der Kondensatoren mittels des Summenladungsmessers durch Messen einer Änderung der Summenladung beim Entladen des jeweiligen Kondensators, sodass Ladungs-Daten erhalten werden, und
(c) Berechnen der Teilchenenergie und/oder einer Strahl-Ladung aus den Ladungs-Daten.

Gemäß einem zweiten Aspekt löst die Erfindung das Problem durch ein Verfahren zum Ermitteln einer Strahlenenergie eines Teilchenstrahls, indem das sukzessive Messen der Ladungen der Kondensatoren dadurch erfolgt, dass der Summenladungsmesser zum Erfassen einer Summenladung aller Ladungen auf den Kondensatoren geschaltet ist, sukzessive alle Kondensatoren entladen werden und die Abnahme der Summenladung für die Entladung jeweils eines Kondensators für die Entladung aller Kondensatoren gemessen wird. Unter dem Merkmal, dass die Abnahme der Summenladung für die Entladung jeweils eines Kondensators für die Entladung aller Kondensatoren gemessen wird, wird insbesondere verstanden, dass die Abnahme der Summenladung für die Entladung jeweils eines Kondensators gemessen wird und dass dies für alle Kondensatoren durchgeführt wird.

Die Erfindung löst das Problem zudem durch ein Verfahren zum Ermitteln einer Strahlenenergie eines Teilchenstrahls mit den Schritten: (a) Einfallen-Lassen des Teilchenstrahls auf ein Teilchenenergie-Messgerät, das (i) zumindest zwanzig Kondensatoren (30.n), die jeweils eine erste Kondensatorplatte und eine zweite Kondensatorplatte aufweisen und bezüglich einer Strahleinfallsrichtung (S) hintereinander angeordnet sind, (ii) einen Multiplexer, der einen Multiplexer-Ausgang und eine Mehrzahl an Multiplexer-Eingängen hat, wobei jeder Multiplexer-Eingang mit genau einem Kondensator verbindbar ist, und der eingerichtet ist zum Verbinden jeweils einer ersten Kondensatorplatte des jeweiligen Kondensators mit dem Multiplexer-Ausgang, (iii) einen Plattenladungsmesser, der einen Plattenladungsmesser-Eingang, der mit den zweiten Kondensatorplatten verbunden ist, und einen Plattenladungsmesser-Ausgang aufweist, (b) sukzessives Messen der Ladungen der Kondensatoren mittels des Plattenladungsmessers durch sukzessives Verbinden der Kondensatoren mit dem Plattenladungsmesser mittels des Multiplexers, sodass Ladungs-Daten erhalten werden, und (c) Berechnen der Teilchenenergie und/oder einer Strahl-Ladung aus den Ladungs-Daten.

Vorteilhaft an der erfindungsgemäßen Lösung ist, dass eine geringe Messunsicherheit erreicht werden kann. Es hat sich herausgestellt, dass insbesondere eine hohe Messgenauigkeit erreicht werden kann, wenn eine große Zahl an Kondensatoren vorhanden ist. So umfasst das Teilchenenergie-Messgerät vorzugsweise 64 Kondensatoren, insbesondere vorzugsweise 100 Kondensatoren. Günstig ist es, wenn höchstens 1000 Kondensatoren vorhanden sind.

Günstig ist zudem, dass sehr geringe Messzeiten erreichbar sind. So ist es möglich, die Teilchenenergie mit einer Messzeit von weniger als einer Minute, insbesondere weniger als zehn Sekunden, insbesondere sogar weniger als einer Sekunde, zu messen. Das erlaubt beispielsweise eine deutlich schnellere Justierung eines Teilchenbeschleunigers.

Günstig ist zudem, dass der Aufwand für die Kalibrierung eines erfindungsgemäßen Teilchenenergie-Messgeräts in der Regel vergleichsweise gering ist. Der Grund dafür ist, dass lediglich ein Ladungsmesser kalibriert werden muss. Das kann mit einer hohen Genauigkeit erfolgen. Theoretisch wäre es möglich, eine Vielzahl an Ladungsmessern vorzusehen, was zu einer noch geringeren Messzeit führen könnte. Der Nachteil daran aber ist, dass der Aufwand zum Kalibrieren der einzelnen Ladungsmesser in diesem Fall sehr groß wird.

Vorteilhaft ist zudem, dass in den meisten Fällen ein gutes Signal-zu-Rausch-Verhältnis erreichbar ist. Je größer die Ladung ist, die sich auf einem einzelnen Kondensator anlagert, desto rauschärmer kann diese Ladung gemessen werden. Es ist daher günstig, den Detektor solange mit dem Teilchenstrahl zu beaufschlagen, bis sich eine hinreichend hohe Zahl an Ladungen auf den Kondensatoren gebildet hat.

Wird kein Summenladungsmesser verwendet, könnte ein zu langes Warten jedoch dazu führen, dass die auf den Kondensatoren gesammelte Ladung so groß wird, dass das resultierende elektrische Feld oberhalb der Durchschlagschwelle liegt, sodass es zu einem Durchschlag kommt. Der Kondensator ist dann zerstört und das Teilchenenergie-Messgerät liefert keine verlässlichen Daten mehr. Wird ein Summenladungsmesser verwendet, kann die Summenladung dazu verwendet werden, den idealen Zeitpunkt für die Auslesung der Kondensatoren zu bestimmen, was eine bevorzugte Ausführungsform eines erfindungsgemäßen Verfahrens darstellt. Das eröffnet die Möglichkeit, den Teilchenstrahls solange auf das Teilchenenergie-Messgerät zu richten, bis eine hinreichend hohe Ladung auf den Kondensatoren vorhanden ist, um ein hohes Signal-zu-Rausch-Verhältnis zu erhalten.

Unter dem Summenladungsmesser wird ein, insbesondere getakteter, Ladungsintegrator verstanden. Der Begriff soll andeuten, dass mit diesem Ladungsintegrator die gesamte Ladung auf allen Kondensatoren gemessen wird. Der Summenladungsmesser könnte auch als erster Integrator bezeichnet werden.

Unter dem Plattenladungsmesser wird ebenfalls ein, insbesondere getakteter, Ladungsintegrator verstanden. Der Begriff soll andeuten, dass mit diesem Ladungsintegrator die Ladung auf den Platten nur eines Kondensators gemessen wird. Der Plattenladungsmesser könnte auch als zweiter Integrator bezeichnet werden.

Unter dem Merkmal, dass das Teilchenenergie-Messgerät ein Teilchenenergie-Messgerät zum Bestimmen der Energie eines Teilchenstrahls ist, wird insbesondere verstanden, dass das Teilchenenergie-Messgerät zum Messen der Energie geeignet ist und vorzugsweise auch die Energie als Messergebnis ausgibt. Günstig ist es, wenn das Teilchenenergie-Messgerät zudem aus gemessenen Größen wie Ladung und Energie auch daraus berechenbare Größen ausgeben kann wie beispielsweise die Strahlleistung, Energiedosis und/oder die Fluenz.

Günstig ist es, wenn die Kondensatoren ein festes Dielektrikum aufweisen. Das feste Dielektrikum ist vorzugsweise eine Oxidschicht, die auf einer oder beiden Kondensatorplatten aufgebracht ist. Ist eine Kondensatorplatte beispielsweise aus Aluminium aufgebaut, ist das Dielektrikum vorzugsweise durch eine eloxierte Schicht gebildet. Danach könnte die eloxierte Schicht metallisiert werden, beispielsweise mit Kupfer, was die zweite Kondensatorplatte bildet.

Es sei für alle Ausführungsformen darauf hingewiesen, dass die Kondensatorplatten nicht voneinander trennbar sein müssen. Es ist auch möglich, dass die Kondensatorplatten miteinander, insbesondere untrennbar, verbunden sind. Insbesondere kann zumindest eine Kondensatorplatte als Beschichtung eines anderen Objekts, beispielsweise der anderen Kondensatorplatte, ausgebildet sein.

Alternativ kann das feste Dielektrikum durch eine Kunststoffplatte gebildet sein. Vorzugsweise enthält der Kunststoff zu über 95 Gew.-% Moleküle, die keine Atome mit einer Kernladungszahl von mehr als neun enthalten. Besonders günstig ist es, wenn die Kunststoffplatten aus halogenfreiem Kunststoff bestehen. Je kleiner die Kernladungszahl ist, desto geringer ist die Wechselwirkung mit Teilchenstrahlen, sodass im Dielektrikum stattfindende Prozesse die Messunsicherheit nur gering beeinflussen. Vorzugsweise bestehen die Kunststoffplatten aus PET (Polyethylenterephthalat).

Besonders günstig ist es, wenn die erste Kondensatorplatte, das feste Dielektrikum und die zweite Kondensatorplatte miteinander zu Kondensatoren verklebt sind. Es ist dann möglich, die einzelnen Kondensatoren aufeinander zu stapeln, sodass das Teilchenenergie-Messgerät vergleichsweise einfach hergestellt werden kann. Es ist dann zudem möglich, einzelne Kondensatoren mit geringem Aufwand auszutauschen, sofern diese defekt sein sollten.

Gemäß einer bevorzugten Ausführungsform umfasst das Teilchenenergie-Messgerät eine Auswerteschaltung, die ausgebildet ist zum automatischen Durchführen eines Verfahrens mit dem Schritt eines Erfassens der Ladung, die vom jeweiligen Kondensator abfließt, durch Bilden der Differenz zwischen der Ladung auf dem Summenladungsmesser zu Beginn und am Ende des Entladens des jeweiligen Kondensators, wobei dieser Schritt für jede Stellung des Multiplexers und damit für alle Kondensatoren durchgeführt wird. Auf diese Weise werden Ladungs-Daten erhalten.

Die Ladungs-Daten kodieren vorzugsweise die Ladung eines jeden Kondensators und enthalten eine Kennung, anhand der der jeweilige Kondensator ermittelbar ist. Diese Kennung kann beispielsweise auch dadurch kodiert sein, dass die Ladungs-Daten in einer vorgegebenen Reihenfolge ausgegeben werden und die Reihenfolge des Auslesens der Kondensatoren konstant und bekannt ist.

Vorteilhafterweise wird dazu der Multiplexer-Ausgang direkt mit der Masse verbunden. Damit werden die erste und die zweite Kondensatorplatte des entsprechenden Kondensators über den Multiplexer und den Summenladungsmesser miteinander verbunden. So ist nur ein einziger Summenladungsmesser, also Integrator, notwendig, was den Kalibrieraufwand reduziert.

Gemäß einer bevorzugten Ausführungsform ist die Auswerteschaltung ausgebildet zum Ansteuern des Multiplexers, sodass dieser die Kondensatoren sukzessive entlädt, auf das Empfangen eines Triggersignals hin. In anderen Worten ist die Auswerteschaltung vorzugsweise ausgebildet zum Ansteuern des Multiplexers, sodass dieser die Kondensatoren sukzessive entlädt, wenn die Auswerteschaltung ein Triggersignal empfängt. Unter einem Triggersignal wird ein Signal verstanden, dass die Tatsache kodiert, dass innerhalb eines vorgegebenen Zeitintervalls um den Zeitpunkt des Triggersignals ein Teilchenstrahl vom Beschleuniger abgegeben wird.

Günstig ist es, wenn das Teilchenenergie-Messgerät (a) einen Plattenladungsmesser hat, der (i) einen Plattenladungsmesser-Eingang, der mit dem Multiplexer-Ausgang verbunden ist, und (ii) einen Plattenladungsmesser-Ausgang aufweist und der (iii) ausgebildet ist zum Messen einer Ladung, die über den Multiplexer-Ausgang und den Plattenladungsmesser-Eingang fließt. Günstig, nicht aber notwendig ist es, wenn die Bezugsmasse des Summenladungsmesser-Ausgangs auf dem gleichen elektrischen Potential liegt wie die des Plattenladungsmesser-Ausgangs.

Günstig ist es, wenn die Auswerteschaltung mit dem Summenladungsmesser und dem Multiplexer verbunden ist und ausgebildet ist zum automatischen Erfassen der von jedem Kondensator abfließenden Ladung durch Erfassen der vom Plattenladungsmesser gemessenen Ladung. Auf diese Weise werden Ladungs-Daten erhalten. Mittels des Plattenladungsmessers kann die Ladung, die auf den Kondensatorplatten vorhanden ist, mit einer besonders hohen Genauigkeit gemessen werden. Es sei darauf hingewiesen, dass es in diesem Fall möglich, nicht aber notwendig ist, dass die Teilchenenergie ausschließlich aus den Lade-Daten berechnet wird, die vom Plattenladungsmesser erfasst wurden. Der Summenladungsmesser kann dann beispielsweise dazu eingesetzt werden, den idealen Zeitpunkt für eine Messung zu ermitteln.

Dazu ist die Auswerteschaltung vorzugsweise ausgebildet zum automatischen sukzessiven Auslesen der Ladungen der Kondensatoren, wenn die vom Plattenladungsmesser gemessene Ladung einen vorgegebenen Schwellenwert überschritten hat.

Das erfolgt, wie oben beschrieben durch das sukzessive Verbinden der Kondensatoren mittels des Multiplexers mit dem Plattenladungsmesser, sodass die vom jeweiligen Kondensator gespeicherte Ladung vom Plattenladungsmesser erfasst und der Kondensator dabei entladen wird.

Gemäß einer bevorzugten Ausführungsform werden der Plattenladungsmesser und der Summenladungsmesser zum Ermitteln der Ladungen der Kondensatoren verwendet. Der Vorteil des Verwendens beider Ladungsmesser liegt in einer verringerten Messunsicherheit. Das gilt besonders, wenn pro Sekunde mehr als eine Messung durchgeführt wird und/oder die Pulsladung eines Teilchenstrahls klein ist. Zwar sind die Ladungen auf den einzelnen Kondensatoren wesentlich kleiner als die Summenladung, vorzugsweise wird aber der Messbereich des Plattenladungsmessers mindesten um den Faktor 10 empfindlicher eingestellt. Die Summe der einzelnen Kanalladungen liefert einen zweiten, unabhängigen Wert für die gesamte Strahl-Ladung, was zu einer Redundanz der Ladungsmessung führt.

Günstig ist es, wenn die Auswerteschaltung ausgebildet ist zum automatischen Entladen der Kondensatoren, wenn nach Vergehen eines vorgegebenen Abschaltzeitraums kein Triggersignal empfangen wird. Auf diese Weise wird die Gefahr minimiert, dass einer der Kondensatoren zu stark geladen wird.

Unter der effektiven Tiefe wird eine Tiefenangabe verstanden, für die gilt, dass zwei Platten unterschiedlicher Dichten und/oder unterschiedlicher Dicken genau dann die gleiche Wirkung auf die Teilchen haben, wenn die effektiven Dicken der beiden Platten gleich sind. Da die Stärke der Interaktion zwischen den Teilchen und dem Material in guter Näherung von dessen Dichte abhängt, ist die effektive Tiefe vorzugsweise das Produkt aus der Dichte und der absoluten Tiefe. Unter der effektiven Tiefe wird insbesondere das Produkt aus der absoluten Tiefe, also einer Länge, und der Dichte des Materials, durch die der Strahl verläuft, verstanden. Verläuft der Teilchenstrahl beispielsweise durch Aluminium, so ist die effektive Tiefe das Produkt aus der Dichte von Aluminium und der Strecke, die der Strahl im Aluminium zurücklegt.

Alternativ oder zusätzlich ist die Auswerteschaltung vorzugsweise ausgebildet zum automatischen Berechnen der Teilchenenergie aus den Ladungs-Daten anhand der Schritte (i) Bestimmen eines Breitenmaßes der Ladungsfunktion und (ii) Bestimmen der Teilchenenergie aus dem Breitenmaß. Unter dem Breitenmaß wird eine Zahl verstanden, die angibt, wie breit die Ladungsfunktion über einer Tiefe, insbesondere über der effektiven Tiefe, ist. Haben die Teilchen eine hohe Teilchenenergie, so können sie noch in größeren effektiven Tiefen ionisieren. Die Breite der Ladungs-Funktion ist daher ein gutes Maß für die Teilchenenergie.

Vorzugsweise erfolgt das Bestimmen des Breitenmaßes durch nummerisches Bestimmen des Intergrals unter der normierten Ladungs-Funktion. Das Normieren bewirkt, dass das Breitenmaß unabhängig wird von der Summenladung, also der Summe aller Ladungen auf den Kondensatoren. Beispielsweise wird das Normieren erreicht durch Teilen durch den Maximalwert der Ladungs-Funktion. Es ist auch möglich, dass die Ladungs-Funktion mit einer Modellfunktion angepasst wird (was auch als Anfitten bezeichnet wird) und dass die Ladungsfunktion zum Normieren dann durch den Maximalwert der ermittelten Ausgleichsfunktion geteilt wird.

Vorzugsweise beträgt eine spezifische Kapazität zumindest für eine Mehrzahl der Kondensatoren 30 bis 100 Pikofarad pro Quadratzentimeter. Alternativ oder zusätzlich beträgt die Kapazität zumindest für eine Mehrzahl der Kondensatoren zumindest 1 Nanofarad, insbesondere zumindest 7 Nanofarad. Auf diese Weise wird ein günstiges Signal-zu-Rausch-Verhältnis ermöglicht. Vorzugsweise ist die Kapazität kleiner als 100 Nanofarad. Das hält den Aufwand zum Herstellen des Kondensators in vertretbarem Rahmen.

Durch die angegebene Kapazität und/oder spezifische Kapazität ist es möglich, die Ladungen in den Kondensatoren zu akkumulieren. Das erleichtert die sequenzielle Auslesung der Kanalladungen mit nur einem Integrator. Bei geringen Plattenkapazitäten müsste vorzugsweise parallel gemessen werden, da bereits geringe Ladungen hohe Spannungen aufbauen. Bei paralleler Auslesung wird die Ladung permanent aus dem Kondensator abgeführt. Bei einer solchen Bauform müsste aber jeder Integrator wie der andere sein, insbesondere auch hinsichtlich des jeweiligen Leckstroms, sonst verrauschen die gemessenen Ladungs-Profile und eine Energiemessung wird dadurch mit einer hohen Messunsicherheit behaftet.

Ein Isolationswiderstand zwischen der ersten Kondensatorplatte und der zweiten Kondensatorplatte beträgt zumindest für eine Mehrzahl der Kondensatoren vorzugsweise zumindest ein Gigaohm. Vorzugsweise gilt dieses Merkmal für zumindest 90 % der Kondensatoren, insbesondere für alle Kondensatoren. Der hohe Widerstand zwischen den Kondensatorplatten eines Kondensators bewirkt, dass während der Messung kein signifikanter Anteil an Ladungen verloren geht. Es ist nicht notwendig, dass der Isolationswiderstand größer als 10 Gigaohm ist.

Um eine geringe Messunsicherheit zu erreichen, ist es günstig, wenn eine Kapazität zumindest für eine Mehrzahl der Kondensatoren der Kondensatoren größer ist, insbesondere um einen Faktor von zumindest 5 größer ist als eine Zwischenkondensator-Kapazität, zwischen Kondensatorplatten benachbarter Kondensatoren.

Es hat sich herausgestellt, dass es günstig ist, wenn zumindest eine Mehrzahl der ersten Kondensatorplatten überwiegend aus Kupfer und/oder Aluminium besteht. Die erste Kondensatorplatte ist vorzugsweise die strahlseitige Kondensatorplatte des entsprechenden Kondensators.

Vorzugsweise hat das Teilchenenergie-Messgerät eine Einstrahlseite. Diese Einstrahlseite ist beispielsweise am Teilchenenergie-Messgerät gekennzeichnet oder in einer Bedienungsanleitung angegeben. Günstig ist es, wenn eine flächenbezogene Dichte der (bezüglich einer Strahleinfallsrichtung S) einstrahlseitigen Kondensatorplatte größer ist als eine flächenbezogene Dichte der strahlabgewandten Kondensatorplatte des Kondensators, vorzugsweise zumindest um den Faktor 2, besonders bevorzugt zumindest um den Faktor 3.

Günstig ist es, wenn für zumindest zwei, insbesondere für zumindest eine Mehrheit der Kondensatoren und/oder Kondensatorplatten gilt, dass eine effektive Dicke der strahlabgewandten Kondensatorplatte kleiner ist als eine effektive Dicke der strahlseitigen Kondensatorplatte. Die effektive Dicke ist die tatsächliche Dicke der Kondensatorplatte multipliziert mit der Dichte des Materials, aus dem die Kondensatorplatte besteht.

Für zumindest zwei Kondensatoren gilt vorzugsweise, dass eine effektive Dicke zumindest einer Kondensatorplatte für den strahlabgewandten Kondensator größer ist als die effektive Dicke zumindest einer Kondensatorplatte des strahlseitigen Kondensators.

Vorzugsweise ist die effektive Dicke der strahlseitigen Kondensatorplatte zumindest doppelt so groß wie die effektive dicke der strahlabgewandten Kondensatorplatte.

Günstig ist es, wenn die strahlseitige Kondensatorplatte eine kleinere Dichte hat als die strahlabgewandte Kondensatorplatte.

Beispielsweise beträgt die effektive Dicke der strahlseitigen Alu-Kondensatorplatte zwischen 0,3 und 3 Gramm pro Kubikzentimeter und die effektive Dicke der strahlabgewandten Kondensatorplatte zwischen 0,15 und 0,35 Gramm pro Kubikzentimeter.

Beispielsweise besteht die strahlseitige Kondensatorplatte aus Graphit oder Aluminium. Eine Dichte der strahlseitigen Kondensatorplatte beträgt vorzugsweise höchstens 3 Gramm pro Kubikzentimeter. Vorzugsweise beträgt auch die Dichte der strahlabgewandten Kondensatorplatte höchstens 3 Gramm pro Kubikzentimeter.

Vorzugsweise gilt für zumindest zwei Kondensatoren, insbesondere zumindest 20, dass eine effektive Dicke zumindest einer Kondensatorplatte für den strahlabgewandten Kondensator größer ist als die effektive Dicke zumindest einer Kondensatorplatte des strahlseitigen Kondensators. Dadurch steigt die Auflösung der Energie bei kleinen Teilchenenergien.

Besonders günstig ist es, wenn für zumindest zwei, insbesondere zumindest 20, Kondensatoren gilt, dass eine effektive Dicke der strahlseitigen Kondensatorplatte für den strahlabgewandten Kondensator größer ist als die effektive Dicke der strahlseitigen Kondensatorplatte des strahlseitigen Kondensators.

Ein erfindungsgemäßes Teilchenenergie-Messgerät besitzt vorzugsweise einen Kalibrierschein, in dem die Messunsicherheit für zumindest eine Teilchenenergie angegeben ist. Insbesondere enthält der der Kalibrierschein vorzugsweise (a) die Messunsicherheit für zumindest eine Teilchenenergie und/oder (b) einen Breiten-Kalibrierfaktor, mittels dem aus dem Breitenmaß die Teilchenenergie berechenbar ist.

Die Auswerteschaltung ist vorzugsweise ausgebildet zum, insbesondere automatischen, Bewirken einer Entladung der Kondensatoren zumindest dann, wenn die Summenladung einen vorgegebenen Ladung-Schwellenwert überschreitet. Das schützt die Kondensatoren vor einer zu großen Spannung. Der Ladung-Schwellenwert entspricht vorzugsweise einer Spannung von 10±1 Volt am Summenladungsmesser.

Günstig ist es, wenn die Auswerteschaltung zudem ausgebildet ist zum automatischen Bewirken einer Entladung der Kondensatoren, wenn (i) die Versorgungsspannung für die Elektronik fehlt, (ii) das Triggersignal fehlt, (iii) eine Trigger-Frequenz zu hoch ist, sodass es zu Puls-Überlappung kommen kann, und/oder (iv) das Integrator-Signal eine Spannung von über 10V hat.

Erfindungsgemäß ist zudem ein Beschleunigersystem mit einem Teilchenbeschleuniger, insbesondere einem Linearbeschleuniger, zum Erzeugen eines monoenergetischen Teilchenstrahls und einem erfindungsgemäßen Messgerät, das angeordnet ist zum Messen einer Teilchenenergie von Teilchen des Teilchenstrahls und/oder einer Strahlladung des Teilchenstrahls. Bei dem Teilchenbeschleuniger handelt es sich vorzugsweise um einen Elektronen- Protonen- oder Ionenbeschleuniger. Die Auswerteschaltung ist vorzugsweise ausgebildet zum automatischen Durchführen eines erfindungsgemäßen Verfahrens. Der Teilchenstrahl besteht vorzugsweise aus geladenen Teilchen, insbesondere ein Elektronen, Protonen oder Ionen. Der Teilchenstrahl kann kontinuierlich sein. Vorzugsweise ist der Teilchenstrahl gepulst.

Vorzugsweise wird ein erfindungsgemäßes Verfahren so durchgeführt, dass ein Teilchenstrahl aus Teilchen mit einer mittleren Teilchenenergie erzeugt wird und ein Teilchenenergie-Messgerät verwendet wird, dessen Kondensatorplatten gemeinsam eine solche Dicke haben, dass eine Sammeleffizienz für die Teilchen der mittleren Teilchenenergie zumindest 95% beträgt. Das bedeutet in anderen Worten, dass höchstens 5 % aller Teilchen das Teilchenenergie-Messgerät durchqueren, ohne dass sie absorbiert wurden.

Vorzugsweise liegt die mittlere Teilchenenergie zwischen 2 Megaelektronenvolt und 1500 Megaelektronenvolt. Besonders bevorzugt beträgt die mittlere Teilchenenergie zwischen 2 MeV und 500 MeV, wenn die Teilchen Elektronen sind. Alternativ oder zusätzlich beträgt die mittlere Teilchenenergie bevorzugt zwischen 20 MeV und 1000 MeV, wenn die Teilchen Photonen oder Ionen sind.

Erfindungsgemäß ist zudem ein Teilchenenergie-Messgerät zum Bestimmen eines Teilchenstrahls mit (a) zumindest zwanzig Kondensatoren, die (i) jeweils eine erste Kondensatorplatte und (ii) eine zweite Kondensatorplatte aufweisen und (iii) bezüglich einer Strahleinfallsrichtung hintereinander angeordnet sind, (b) einem Multiplexer, der (i) einen Multiplexer-Ausgang und (ii) eine Mehrzahl an Multiplexer-Eingängen hat, wobei jeder Multiplexer-Eingang mit genau einem Kondensator verbunden ist, und der (iii) eingerichtet ist zum Verbinden jeweils einer ersten Kondensatorplatte des jeweiligen Kondensators mit dem Multiplexer-Ausgang, (c) einem Plattenladungsmesser, der (i) einen Plattenladungsmesser-Eingang, der mit dem Multiplexer-Ausgang verbunden ist, und (ii) einen Plattenladungsmesser-Ausgang aufweist und (iii) ausgebildet ist zum Messen einer Ladung, die über den Multiplexer-Ausgang und den Plattenladungsmesser fließt, (d) einer Auswerteschaltung, die mit dem Plattenladungsmesser und dem Multiplexer verbunden ist und ausgebildet ist zum automatischen (i) Bewirken eines Umschaltens von einem Multiplexer-Eingang auf einen anderen Multiplexer-Eingang, sodass sukzessive die Kondensatoren einzeln entladen werden, und (ii) Erfassen der von jedem Kondensator beim Entladen abfließenden Ladung, sodass Ladungs-Daten erhalten werden, aus denen die Teilchenenergie berechenbar ist.

Es ist möglich, nicht aber notwendig, dass dieses Teilchenenergie-Messgerät einen Summenladungsmesser aufweist, der einen Summenladungsmesser-Eingang, der mit den zweiten Kondensatorplatten verbunden ist und ausgebildet zum Erfassen einer Summenladung der Kondensatoren und einen Summenladungsmesser-Ausgang aufweist. Die oben beschriebenen bevorzugten Ausführungsformen für Teilchenenergie-Messgeräte gelten auch für diese Erfindung.

Erfindungsgemäß ist zudem ein Teilchenenergie-Messgerät (a) zumindest zwanzig Kondensatoren, die (i) jeweils eine erste Kondensatorplatte und (ii) eine zweite Kondensatorplatte aufweisen und (iii) bezüglich einer Strahleinfallsrichtung hintereinander angeordnet sind, (b) einem Multiplexer, der (i) einen Multiplexer-Ausgang und (ii) eine Mehrzahl an Multiplexer-Eingängen hat, wobei jeder Multiplexer-Eingang mit genau einem Kondensator verbunden ist, und der (iii) eingerichtet ist zum Verbinden jeweils einer ersten Kondensatorplatte des jeweiligen Kondensators mit dem Multiplexer-Ausgang, (c) einem Plattenladungsmesser, der (i) einen Plattenladungsmesser-Eingang, der mit dem Multiplexer-Ausgang verbunden ist, und (ii) einen Plattenladungsmesser-Ausgang aufweist und (iii) ausgebildet ist zum Messen einer Ladung, die über den Multiplexer-Ausgang und den Plattenladungsmesser-Ausgang fließt, und (d) einem Summenladungsmesser, der (i) einen Summenladungsmesser-Eingang, der mit den zweiten Kondensatorplatten verbunden ist zum Erfassen einer Summenladung der Ladungen auf allen Kondensatoren, und (ii) einen Summenladungsmesser-Ausgang aufweist.

Es ist möglich, nicht aber notwendig, dass dieses Teilchenenergie-Messgerät eine Auswerteschaltung aufweist, die zum automatischen (i) Bewirken eines Umschaltens von einem Multiplexer-Eingang auf einen anderen Multiplexer-Eingang eingerichtet ist, sodass sukzessive die Kondensatoren einzeln entladen werden, und (ii) Erfassen der von jedem Kondensator beim Entladen abfließenden Ladung, sodass Ladungs-Daten erhalten werden, aus denen die Teilchenenergie berechenbar ist. Die oben angegebenen bevorzugten Ausführungsformen für ein Teilchenenergie-Messgerät gelten auch für diese Erfindung.

Die Erfindung wird anhand der beigefügten Zeichnungen erläutert. Dabei zeigt
- Figur 1: ein erfindungsgemäßes Beschleunigungssystem mit einem erfindungsgemäßen Teilchenenergie-Messgerät,
- Figur 2a: eine perspektivische Ansicht eines erfindungsgemäßen Teilchenenergie-Messgeräts,
- Figur 2b: eine Detailansicht eines Kondensators des Teilchenenergie-Messgeräts gemäß Figur 2a,
- Figur 3: ein schematisches Schaltbild eines erfindungsgemäßen Teilchenenergie- Messgeräts gemäß einer ersten Ausführungsform,
- Figur 4: ein schematisches Schaltbild eines erfindungsgemäßen Teilchenenergie-Messgeräts gemäß einer zweiten Ausführungsform,
- Figur 5: ein schematisches Schaltbild eines erfindungsgemäßen Teilchenenergie-Messgeräts gemäß einer dritten Ausführungsform,
- Figur 6a: ein Diagramm, in dem die auf den Kondensatoren vorhandene Ladung gegen die jeweilige effektive Tiefe aufgetragen ist,
- Figur 6b: ein Diagramm, in dem die Breite der Kurve gemäß Figur 6a über die Teilchenenergie aufgetragen ist,
- Figur 7: ein Blockschaltbild einer Auswerteeinheit des Teilchenenergie-Messgeräts und
- Figur 8: ein Schaltbild des Plattenladungsmessers.

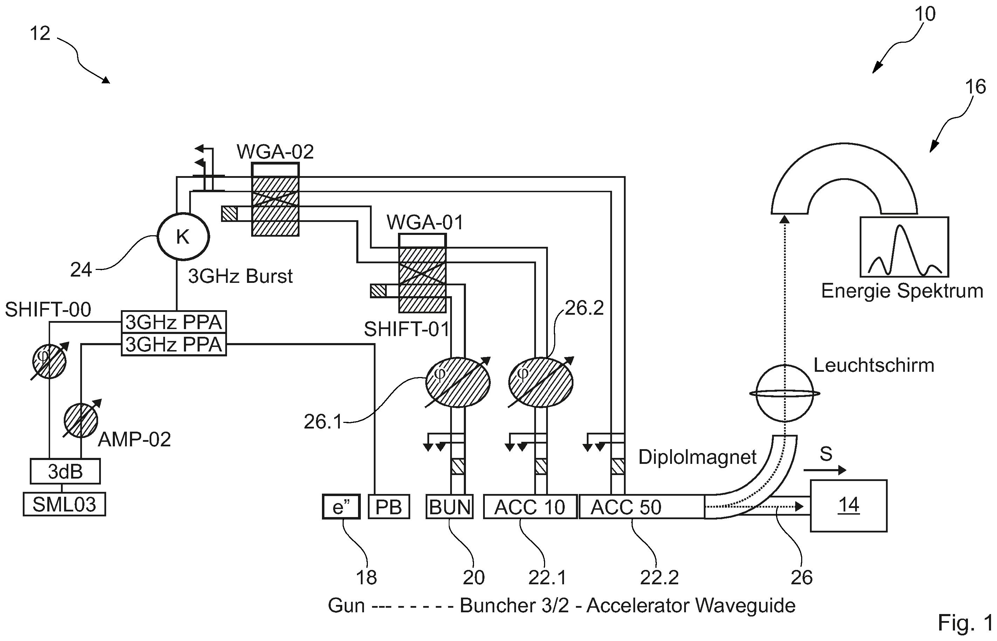

Figur 1 zeigt ein Beschleunigersystem 10, das einen Teilchenbeschleuniger 12, ein erfindungsgemäßes Teilchenenergie-Messgerät 14 sowie, was aber optional ist, ein Spektrometer 16, aufweist. Das Spektrometer 16 wird lediglich zum Kalibrieren des Teilchenenergie-Messgeräts 14 verwendet und ist daher beim Einsatz des Beschleunigersystems 10 nach dem Kalibrieren entbehrlich. Mittels des Teilchenenergie-Messgeräts 14 kann der Teilchenbeschleuniger 12 schnell justiert werden. Nach der Justage kann das Teilchenenergie-Messgerät 14 entfernt werden. Der Teilchenbeschleuniger 12 ist dann einsatzbereit.

Vorzugsweise handelt es sich bei dem Teilchenbeschleuniger 12 um einen Elektronen- oder Protonenbeschleuniger. Der Teilchenbeschleuniger 12 umfasst eine Teilchenquelle 18, beispielsweise eine Elektronenquelle, einen Strahlformer 20 sowie zwei Beschleuniger-Einheiten 22.1, 22.2. Die Beschleuniger-Einheiten 22.1, 22.2 werden von einem Klystron 24 mit einer HF-Wechselspannung beaufschlagt, die eine Frequenz von beispielsweise F = 3 Ghz haben kann. Frequenzen zwischen 1 und 5 Ghz sind möglich. Über die Phasenschieber 26.1, 26.2 können der an den Buncher 20 und an die erste Beschleuniger-Struktur 22.1 eingekoppelte RF-Leistung Phasenverschiebungen *φ*₁, *φ*₂ aufgeprägt werden. Eine Beschleunigungsspannung liegt bei beispielsweise U = 50 Megavolt. Die Beschleunigungsspannung ist mindestens so hoch wie die gewünschte Teilchenenergie. Sie ist eine zeitlich veränderliche Größe und entsteht an der Serienresonanz der Beschleuniger-Struktur mit Hilfe der RF-Leistung des Klystrons. Dabei spielt die Resonanzgüte eine Rolle, denn es gilt: U = Q √(P _{*} Z) . Eine 3GHz-Struktur erreicht eine Resonanzgüte Q von bis zu 20000. Teilchenbeschleuniger 12 sind in der Literatur gut bekannt und werden daher nicht weiter beschrieben.

Der Teilchenbeschleuniger 12 erzeugt einen Teilchenstrahl 26. Günstig ist es, wenn der Teilchenbeschleuniger 12 einen monoenergetischen Teilchenstrahl abgibt. Dazu wird vorzugsweise aus einem nicht-monoenergetischen Teilchenstrahl durch Selektion mittels Energieschlitzen nach einem Auffächern durch einen Dipolmagneten ein monoenergetischer Teilchenstrahl erzeugt. Unter einem monoenergetischen Teilchenstrahl wird ein Teilchenstrahl verstanden, bei dem die einzelnen Teilchen kaum von der mittleren Teilchenenergie abweichen. Beispielsweise beträgt in der Praxis die Abweichung höchstens ± 5 %, vorzugsweise höchstens ± 0,5 %.

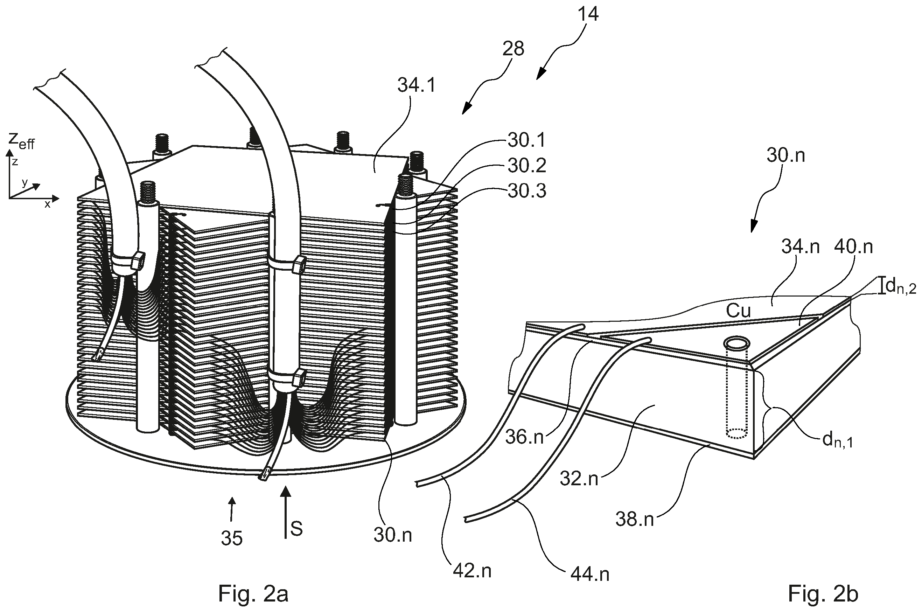

Figur 2a zeigt eine Kondensatoreinheit 28 des Teilchenenergie-Messgeräts 14 gemäß Figur 1. Die Kondensatoreinheit 28 umfasst eine Mehrzahl an Kondensatoren 30.n (n = 1, 2, ..., N). Die Zahl N an Kondensatoren beträgt vorzugsweise zumindest 20, insbesondere zumindest 60. Jeder einzelne Kondensator 30.n besitzt, wie Figur 2b zeigt, eine erste Kondensatorplatte 32.n, eine zweite Kondensatorplatte 34.n und ein dazwischen angeordnetes festes Dielektrikum 36.n, bei dem es sich vorzugsweise um eine Kunststoffplatte handelt. Beispielsweise besteht die Kunststoffplatte aus Polyethylenterephthalat. Die Kondensatorplatten 32.n, 34.n und das Dielektrikum 36.n sind miteinander verklebt. Günstig ist es, wenn der Kondensator 30 wie im vorliegenden Fall einen Isolator 38.n besitzt, der im vorliegenden Fall ebenfalls durch eine Kunststoffplatte gebildet ist. Diese Kunststoffplatte ist mit der ersten Kondensatorplatte 32.n verklebt. Durch Übereinanderstapeln der Kondensatoren 30.n und durch das nachfolgende Kontaktieren ergibt sich die Kondensatoreinheit 28.

Figur 2b zeigt eine besonders einfache Art der Kontaktierung eines Kondensators 30.n. Die erste Kondensatorplatte 32.n ist mit einem Kontaktereich 40.n elektrisch verbunden (durchkontaktiert). Der Grund dafür ist, dass sich Aluminium nicht ohne weiteres löten lässt. Durch einen Kontaktbereich 40.n aus einem gut lötbaren Metall, beispielsweise Kupfer, lässt sich das Problem lösen. Der Kontaktbereich 40.n erstreckt sich in der gleichen Ebene wie die zweite Kondensatorplatte 34.n. Somit ist die erste Kondensatorplatte 32.n mit dem Kabel 44.n und die zweite Kondensatorplatte 34.n mit dem zweiten Kabel 42.n elektrisch verbunden.

Die erste Kondensatorplatte 32.n besteht aus einem ersten Material, das eine erste Dichte *ρ*_{*n*,1} hat. Die zweite Kondensatorplatte 34.n besteht aus einem Material mit einer Dichte *ρ*_{*n*,2}*.* Es ist grundsätzlich möglich, dass sich die Dichten verschiedener Kondensatorplatten 32.a, 32.b (a ≠ b) bzw. 34.a, 34.b (a ≠ b) voneinander unterscheiden. Eine besonders einfache Fertigung wird jedoch erreicht, wenn die jeweiligen Materialen gleich sind, sodass auch die Dichten gleich sind.

Die Dichte *ρ*_{*n,*2} der zweiten Kondensatorplatte ist vorzugsweise größer als die Dichte *ρ*_{*n*,1} der ersten Kondensatorplatte. Beispielsweise besteht die erste Kondensatorplatte 32.n aus Aluminium oder einer Aluminiumlegierung, die zweite Kondensatorplatte 34.n aus Kupfer oder einer Kupferlegierung.

Die erste Kondensatorplatte hat eine Dicke d_{n,1}, die zweite Kondensatorplatte hat eine Dicke d_{n,2}. Günstig ist, wenn die Dicke d_{n,1} der ersten Kondensatorplatte 32.n größer ist als die Dicke d_{n,2} der zweiten Kondensatorplatte 34.n. Vorzugsweise ist die erste Kondensatorplatte 32 die strahlzugewandte Kondensatorplatte, das heißt, dass die Kondensatorplatte 32.n bezüglich einer Strahleinfallsrichtung S vor der zweiten Kondensatorplatte 34.n liegt. In Strahleinfallsrichtung S vorne ist eine Einstrahlseite 35.

Eine effektive Dicke D_{n,1}=*ρ*_{n,1,1} · *d*_{*n*,1} der ersten Kondensatorplatte 32.n ist ebenfalls größer als die effektive Dicke d_{n,2} der zweiten Kondensatorplatte 34.n. Es ist möglich, nicht aber notwendig, dass dies für alle Kondensatoren 30.n gilt.

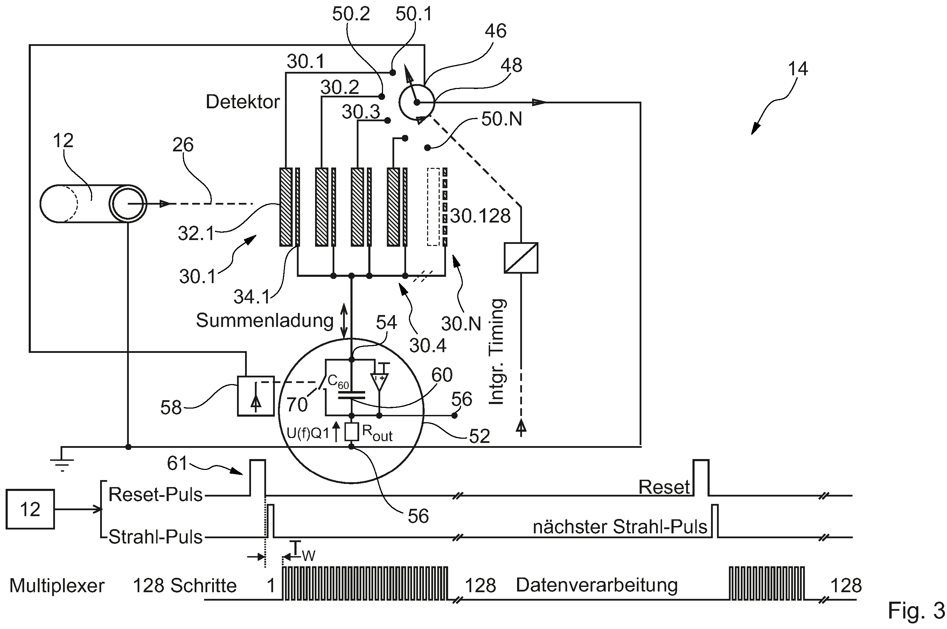

Figur 3 zeigt ein Schaltbild des erfindungsgemäßen Teilchenenergie-Messgeräts 14 mit den Kondensatoren 30.n. Das Teilchenenergie-Messgerät 14 besitzt zudem einen Multiplexer 46, der einen Multiplexer-Ausgang 48 und eine Mehrzahl an Multiplexer-Eingängen 50.n. hat. Jeder Multiplexer-Eingang 50.n ist zum Verbinden mit genau einem Kondensator 30.n ausgebildet.

Der Teilchenstrahl 26 trifft auf die Kondensatoren 30.n und erzeugt dort Ladungen Qₙ.

Das Teilchenenergie-Messgerät 14 besitzt einen Summenladungsmesser 52, der einen Summenladungsmesser-Eingang 54 und einen Summenladungsmesser-Ausgang 56 hat. Der Summenladungs-Eingang 54 ist mit den zweiten Kondensatorplatten 34.n verbunden. Ist der Multiplexer-Eingang 50.n mit dem jeweiligen Kondensator 30.n verbunden, so können Ladungen über den Summenladungsmesser-Eingang 54 und den Multiplexer-Ausgang 48 fließen, sodass die Ladung von dem Kondensator 30 abfließt. Die Spannung Uₙ, die am n-ten Kondensator 30.n anliegt, reduziert sich damit auf null oder näherungsweise null. Unter dem Merkmal, dass sich die Spannung Uₙ auf näherungsweise null reduziert, wird insbesondere verstanden, dass die etwaig verbleibende Spannung so klein ist, dass sich die Messunsicherheit um relativ 10 % erhöht. Eine Kapazität C1 eines Kondensators 60 des Summenladungsmessers 52 beträgt vorzugsweise C1 = 1nF bis C1 = 10 µF.

Figur 3 zeigt zudem, dass das Teilchenenergie-Messgerät 14 eine Auswerteschaltung 58 aufweist, die mit dem Multiplexer 56 zum Umschalten der Multiplexer-Eingänge 50.n verbunden ist. Die Auswerteschaltung 58 ist zudem mit dem Summenladungsmesser 52 verbunden und registriert automatisch die Veränderung einer Summenladung *Q*_{Σ}, die im Summenladungsmesser 52 gespeichert ist. Insbesondre wird die Summenladung *Q*_{Σ} auf einem Kondensator 60 des Summenladungsmessers 52 erfasst. Wird ein vorgegebener Kondensator 30.n entladen, ändert sich die Summenladung *Q*_{Σ} dabei um eine Differenz Δ*Q*_{Σ}. Diese Differenz Δ*Q*_{Σ} = Qₙ entspricht der Ladung Qₙ, die auf den jeweiligen Kondensator 30.n gespeichert war.

Empfängt die Auswerteschaltung 58 ein Triggersignal 61 vom Teilchenbeschleuniger 12 (vgl. Fig. 1), so wird der Summenladungsmesser durch Kurzschließen des Kondensators 60 zurückgesetzt und erfasst während der Wartezeit Tw die nächste Summenladung *Q*_{Σ} auf dem Summenladungsmesser 52. Während der Wartezeit und bis nach dem Auslesen der Summenladung Q_{S} ist der Multiplexer-Eingang 50 mit keinem Kondensator 30.n verbunden.

Nach Ablauf der Wartezeit T_{W} verbindet die Auswerteschaltung 58 automatisch den Multiplexer-Eingang 50.1 sukzessive mit den ersten Kondensatorplatten 32.n der Kondensatoren 30n. Nach dem Verbinden mit einem Kondensator 30.n wird die Veränderung Qₙ = Δ*Q*_{S}, also die Veränderung der Summenladung Q_{S} aufgrund des Entladens des jeweiligen Kondensators 30.n, gemessen. Die entsprechende Ladungsinformation stellt Ladungs-Daten dar, die die jeweiligen Ladungen Qₙ auf dem n-ten Kondensator kodieren. Daraus wird, wie weiter unten erläutert wird, die Teilchenenergie bestimmt.

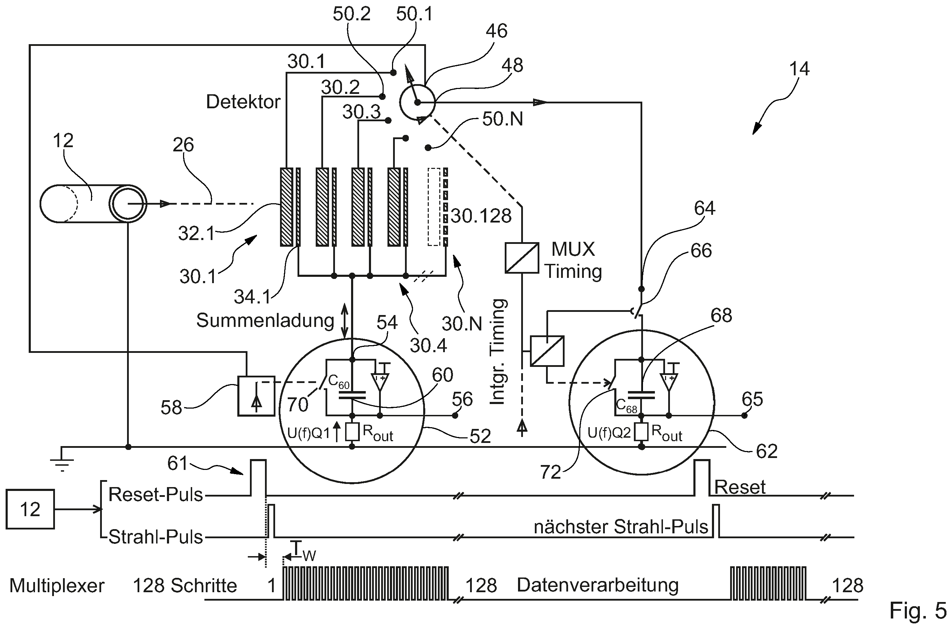

Figur 5 zeigt eine zweite Ausführungsform eines erfindungsgemäßen Teilchenenergie-Messgeräts 14, das einen Plattenladungsmesser 62 besitzt. Der Plattenladungsmesser 62 hat einen Plattenladungsmesser-Eingang 64, der über einen ersten Schalter 66 mit dem Multiplexer-Ausgang 48 verbindbar ist, und einen Plattenladungsmesser-Ausgang 65. Ein Messbereich-Kondensator 68 des Plattenladungsmessers 62 kann über einen zweiten Schalter 72 des Plattenladungsmessers 62 überbrückt werden, sodass sich der Kondensator 68 entlädt. Die Kapazität C₆₈ richtet sich nach der zu erwarteten Plattenladung Qₙ. Sie ist als eine Kombination aus mehreren Messbereichs-Kondensatoren frei umschaltbar und wird so gewählt, dass die Spannung U=Q/C am Ausgang des Plattenladungsmessers 62 eine Schwelle von 10 Volt nicht überschreitet.

Der Plattenladungsmesser 62 ist mit der Auswerteschaltung 58 verbunden, wobei diese Verbindung aus Gründen der Übersichtlichkeit nicht eingezeichnet ist. Überschreitet die Summenladung *Q*_{Σ} einen vorgegebenen Schwellenwert *Q*_{Σ,max}, so steuert die Auswerteschaltung 58 den Multiplexer 46 so an, dass dieser sukzessive die Kondensatoren 30.n mit dem Plattenladungsmesser 62 verbindet.

Bei geschlossenem Schalter 66 und geöffnetem Schalter 72 erfolgt ein Ladungsausgleich am jeweiligen Plattenkondensator 30.n. Die für den Ausgleich benötigte Ladung Qₙ wird vom Plattenladungsmesser 62 erfasst und erscheint an seinem Ausgang 65 als die zu dieser Ladung äquivalente Spannung. Diese Spannung wird von der Auswerteschaltung 58 digitalisiert.

Danach wird der Schalter 66 wieder geöffnet, der Schalter 72 geschlossen und der Multiplexer 46 mit dem nächsten Plattenkondensator 30.n+1 verbunden. Der Zyklus wiederholt sich N-mal bis die Ladung am letzten Plattenkondensator 30.N ausgeglichen und somit gemessen wird. Das Schließen des Schalters 72 entlädt jedes Mal den Messbereichskondensator 68. Das Öffnen des Schalters 66 unmittelbar zuvor verhindert den Rückfluss der Ladung in Richtung Multiplexer 46.

Die Auswerteschaltung ist vorzugsweise ausgebildet zum automatischen Schließen der Schalter 66, 70, 72, wenn das Triggersignal 61 für zumindest einen vorgegebenen Abschalt-Zeitraum ausbleibt.

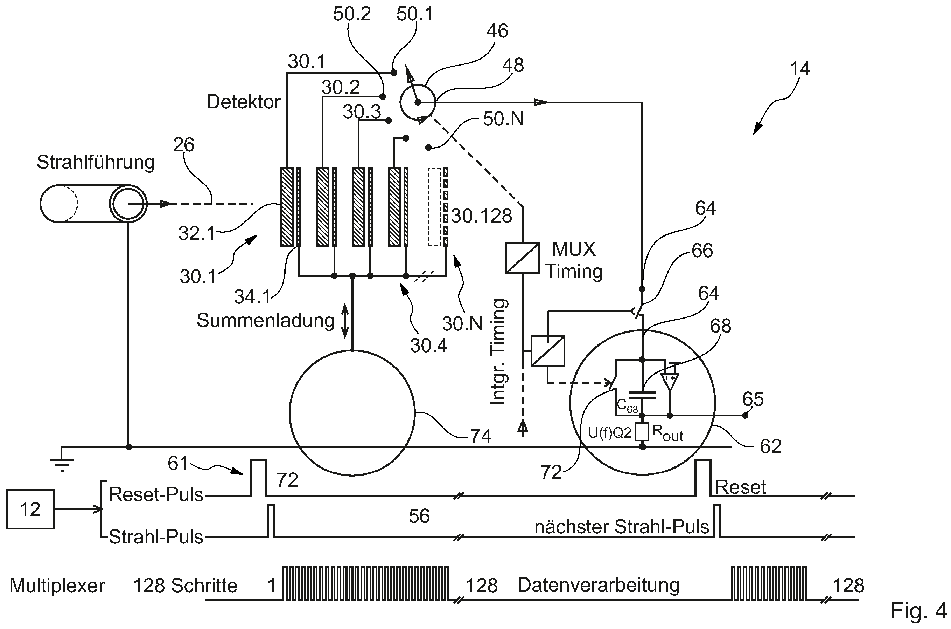

Figur 4 zeigt eine weitre Ausführungsform eines erfindungsgemäßen Teilchenenergie-Messgeräts 14, das keinen Summenladungsmesser 52 besitzt, sondern stattdessen nur eine Sicherheitsschaltung 74, die alle Kondensatoren 30.n kurzschließt, wenn ein Schwellenwert für die Summenladung überschritten wird.

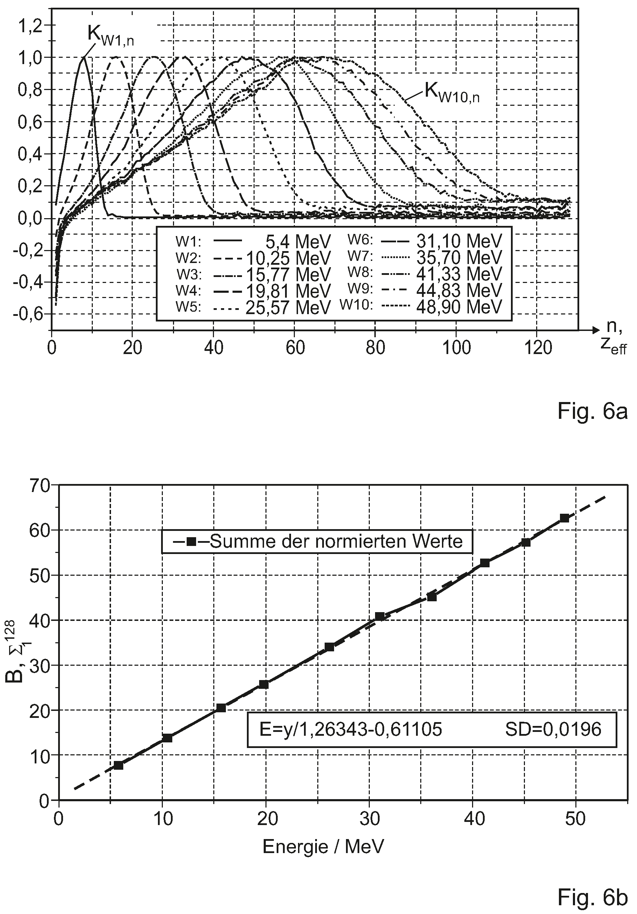

Figur 6a zeigt ein Diagramm, in dem die Ladung Qₙ des n-ten Kondensators auf der Ordinate über dem jeweiligen Kondensator 30.n als Abszisse aufgetragen ist. Im vorliegenden Fall sind alle Kondensatoren 30.n gleich aufgebaut, sodass der Laufindex n der Kondensatoren zugleich eine effektive Tiefe darstellt.

In Figur 6a sind normierte Ladungs-Funktionen K_{W} eingezeichnet, die sich auf die jeweilige Energie W beziehen. Die Ladungskurven K_{W} sind normiert, das heißt, dass ihr maximaler Wert 1 ist.

Figur 6b zeigt die Abhängigkeit einer Breite B der jeweiligen Ladungskurve Kw von der (mittleren) Teilchenenergie E. Die Breite B ist berechnet als das numerische Intergral über die normierte Ladungskurve K_{W}. Anhand eines Breiten-Kalibrierfaktors k in Form des Proportionalitätsfaktors in der Gleichung W = k_{*}B + b und eines Achsabschnitts b, kann aus jeder Breite B die Teilchenenergie berechnet werden. Im vorliegenden Fall gilt für den Breiten-Kalibrierfaktors k = 1,2634 und für den Achsabschnitt b = 0,61105.

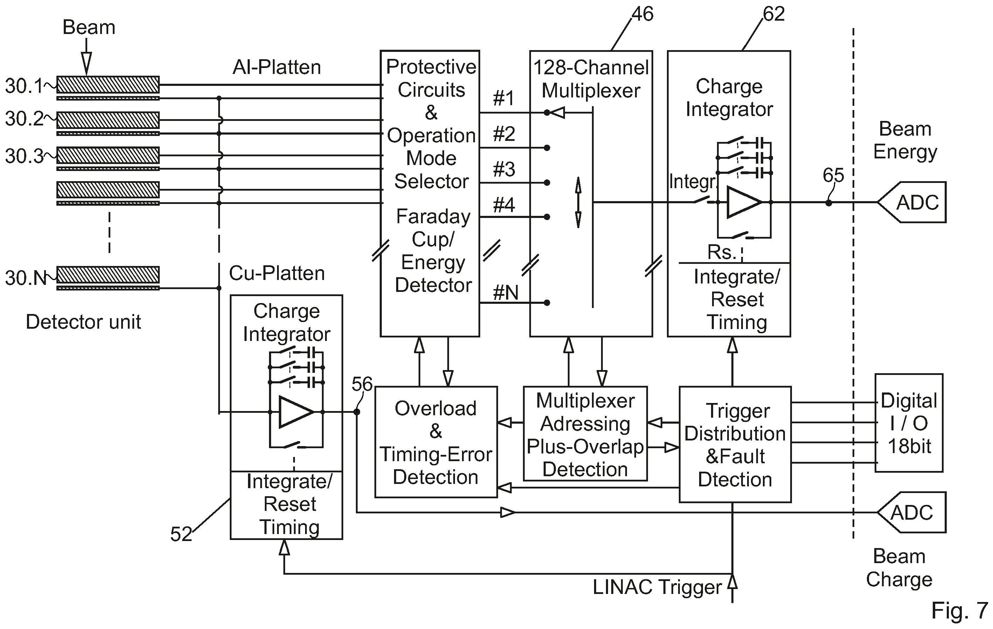

Figur 7 zeigt ein Block-Schaltbild der Auswerteschaltung 58.

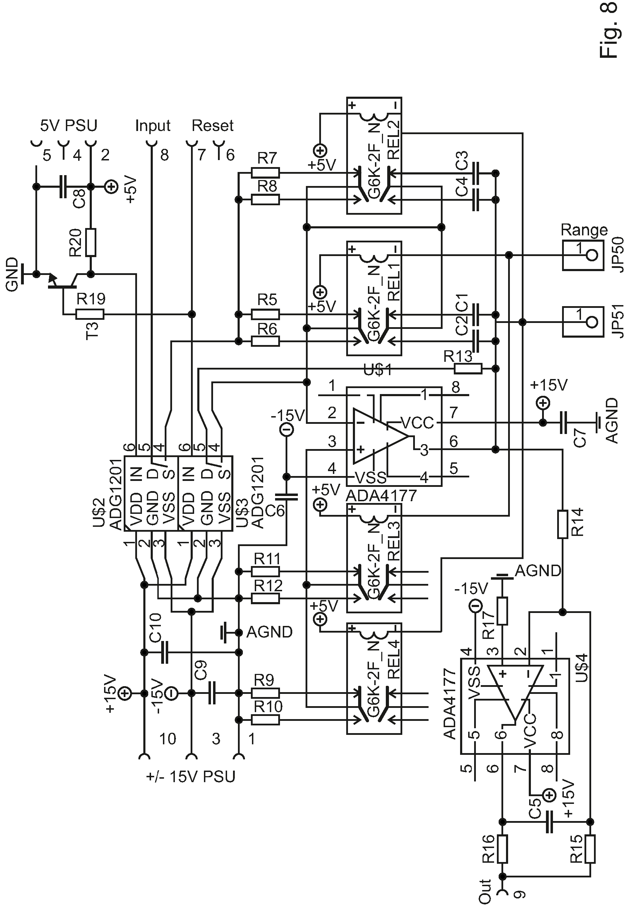

Figur 8 zeigt ein Schaltbild des zweiten Ladungsmessers 62. Die Schaltung des Summenladungsmessers 52 ist im Wesentlichen identisch, es sind lediglich die Pins 4-5 vom Bauteil U$2 überbrückt, weil der Summenladungsmesser den Eingangs-Schalter nicht benötigt.

**Bezugszeichenliste**

| | | | |
|---|---|---|---|
| 10 | Beschleunigersystem | 60 | Kondensator |
| 12 | Teilchenbeschleuniger | 61 | Triggersignal |
| 14 | Teilchenenergie-Messgerät | 62 | Plattenladungsmesser |
| 16 | Spektrometer | 64 | Plattenladungsmesser-Eingang |
| 18 | Teilchenquelle | 65 | Plattenladungsmesser-Ausgang |
| | | 66 | Schalter |
| 20 | Strahlformer | 68 | zweiter Kondensator |
| 22 | Beschleunigereinheit | | |
| 24 | Klystron | 70 | zweiter Schalter |
| 26 | Teilchenstrahl | 72 | dritter Schalter |
| 28 | Kondensatoreinheit | 74 | Sicherheitsschaltung |
| 30 | Kondensator | b | Achsabschnitt |
| 32 | erste Kondensatorplatte | B | Breite |
| 34 | zweite Kondensatorplatte | C | Kapazität |
| 35 | Einstrahlseite | Δ*Q*_{Σ} | Differenz |
| 36 | Dielektrikum | d_{n,1} | Dicke der ersten Kondensatorplatte des n-ten Kondensators |
| 38 | Isolator | | |
| | | D_{n,1} | effektive Dicke erste Kondensatorplatte des n-ten Kondensators |
| 40 | Kontaktbereich | | |
| 41 | Verbinder | | |
| 42 | erstes Kabel | K_{w} | Ladungs-Funktion |
| 44 | zweites Kabel | n | Laufindex |
| 46 | Multiplexer | N | Zahl an Kondensatoren |
| 48 | Multiplexer-Ausgang | Qₙ | Ladung auf dem n-ten Kondensator |
| | | *Q*_{∑} | Summenladung |
| 50 | Multiplexer-Eingang | *ρ*_{*n*,1} | Dichte der ersten Kondensatorplatte |
| 52 | Summenladungsmesser | | |
| 54 | Summenladungsmesser-Eingang | S | Strahleinfallsrichtung |
| 56 | Summenladungsmesser-Ausgang | T_{W} | Wartezeit |
| 58 | Auswerteschaltung | Uₙ | Spannung an n-tem Kondensator |
| | | E | Teilchenenergie |

## Patentansprüche

1. Teilchenenergie-Messgerät (14) zum Bestimmen der Energie eines Teilchenstrahls (26), mit
(a) zumindest zwanzig Kondensatoren (30), die
(i) jeweils eine erste Kondensatorplatte (32) und
(ii) eine zweite Kondensatorplatte (34) aufweisen und
(iii) bezüglich einer Strahleinfallsrichtung (S) hintereinander angeordnet sind,
(b) einem Multiplexer (46), der
(i) einen Multiplexer-Ausgang (48) und
(ii) eine Mehrzahl an Multiplexer-Eingängen (50.n) hat, wobei jeder Multiplexer-Eingang (50) mit genau einem Kondensator (30.n) verbunden ist, und der
(iii) eingerichtet ist zum Verbinden jeweils einer ersten Kondensatorplatte (32.n) des jeweiligen Kondensators mit dem Multiplexer-Ausgang (48),
(a) einem Plattenladungsmesser (62), der
(i) einen Plattenladungsmesser-Eingang (64), der mit dem Multiplexer-Ausgang (48) verbunden (48) ist, und
(ii) einen Plattenladungsmesser-Ausgang (65) aufweist und
(iii) ausgebildet ist zum Messen einer Ladung (Qₙ), die über den Multiplexer-Ausgang (48) und den Plattenladungsmesser-Eingang (64) fließt,
(c) einer Auswerteschaltung (58), die
mit dem Plattenladungsmesser (62) und dem Multiplexer (46) verbunden ist und ausgebildet ist zum automatischen
(i) Bewirken eines Umschaltens von einem Multiplexer-Eingang (50.n) auf einen anderen Multiplexer-Eingang (50.n), sodass sukzessive die Kondensatoren (30.n) einzeln entladen werden, und
(ii) Erfassen der von jedem Kondensator (30.n) beim Entladen fließenden Ladung (Qₙ), sodass Ladungs-Daten erhalten werden, aus denen die Teilchenenergie (E) berechenbar ist.

2. Teilchenenergie-Messgerät (14) nach Anspruch 1, **gekennzeichnet durch** einen Summenladungsmesser (52), der
(i) einen Summenladungsmesser-Eingang (54), der mit den zweiten Kondensatorplatten (34.n) verbunden ist ausgebildet ist zum Erfassen einer Summenladung (*Q*_{Σ}) der Kondensatoren (30.n), und
(ii) einen Summenladungsmesser-Ausgang (56) aufweist.

3. Teilchenenergie-Messgerät (14) nach Anspruch 1 oder 2, **dadurch gekennzeichnet, dass** die Auswerteschaltung (58) ausgebildet ist zum automatischen Durchführen des folgenden Schritts:
(i) für jede Stellung des Multiplexers (46) Erfassen der Ladung (Qₙ), die vom jeweiligen Kondensator (30.n) abfließt, durch Bilden der Differenz (Δ*Q*_{Σ}) zwischen der Ladung auf dem Summenladungsmesser (52) zu Beginn und am Ende des Entladens des jeweiligen Kondensators (30.n), sodass Ladungs-Daten erhalten werden,
(ii) wobei die Ladungs-Daten die Ladung (Qₙ) eines jeden Kondensators (30.n) und der jeweiligen Kondensatoren (30.n.) kodieren.

4. Teilchenenergie-Messgerät (14) nach einem der vorstehenden Ansprüche, **dadurch gekennzeichnet, dass**
die Auswerteschaltung (58) ausgebildet ist zum Ansteuern des Multiplexers (46), sodass dieser die Kondensatoren (30.n) sukzessive entlädt, auf das Empfangen eines Triggersignals (61) hin.

5. Teilchenenergie-Messgerät (14) nach einem der vorstehenden Ansprüche,
**dadurch gekennzeichnet, dass**
die Auswerteschaltung (58) ausgebildet ist zum automatischen sukzessiven Auslesen der Ladungen (Qₙ) der Kondensatoren (30.n), wenn die vom Plattenladungsmesser (62) gemessene Ladung einen vorgegebenen Schwellenwert überschritten hat.

6. Teilchenenergie-Messgerät (14) nach einem der vorstehenden Ansprüche,
**dadurch gekennzeichnet, dass** die Auswerteschaltung (58) ausgebildet ist zum automatischen Entladen der Kondensatoren (30.n), wenn für einen vorgegebenen Abschalt-Zeitraum kein Triggersignal (61) empfangen wird.

7. Teilchenenergie-Messgerät (14) nach einem der vorstehenden Ansprüche, **dadurch gekennzeichnet, dass** die Auswerteschaltung (58) ausgebildet ist zum automatischen Berechnen der Teilchenenergie (E) aus den Ladungs-Daten mittels der folgenden Schritte:
(i) Bestimmen eines Breitenmaßes (B) der Ladungs-Funktion (Kw) und
(ii) Bestimmen der Teilchenenergie (E) aus dem Breitenmaß (B).

8. Teilchenenergie-Messgerät (14) nach einem der vorstehenden Ansprüche, **dadurch gekennzeichnet, dass** zumindest für eine Mehrzahl der Kondensatoren (30.n)
(a) eine spezifische Kapazität (C) 30 bis 100 Pikofarad pro Quadratzentimeter beträgt und/oder
(b) eine Kapazität (Cn) zumindest 1 Nanofarad, insbesondere zumindest 7 Nanofarad, beträgt und/oder höchstens 100 Nanofarad beträgt und/oder
(c) ein Isolationswiderstand zwischen der ersten Kondensatorplatte (32.n) und der zweiten Kondensatorplatte (34.n) zumindest 1 GΩ beträgt, und/oder
(d) eine Kapazität (Cn) des Kondensators (30.n) größer, insbesondere zumindest um einen Faktor 5 größer, ist als eine Zwischenkondensator-Kapazität zwischen Kondensatorplatten benachbarter Kondensatoren.

9. Teilchenenergie-Messgerät (14) nach einem der vorstehenden Ansprüche, **dadurch gekennzeichnet, dass** die Kondensatoren (30.n) ein festes Dielektrikum aufweisen .

10. Teilchenenergie-Messgerät (14) nach einem der vorstehenden Ansprüche,
**dadurch gekennzeichnet, dass**
(a) das Teilchenenergie-Messgerät (14) eine Einstrahlseite (35) hat und
(b) eine flächenbezogene Dichte (*ρ*ₙ) der einstrahlseitigen Kondensatorplatte (32.n) zumindest das Fünffache einer flächenbezogenen Dichte (*ρ*ₙ₁) der strahlabgewandten Kondensatorplatte des Kondensators beträgt.

11. Teilchenenergie-Messgerät (14) nach einem der vorstehenden Ansprüche,
**dadurch gekennzeichnet, dass**
zumindest für eine Mehrheit der Kondensatoren (30.n) gilt, dass eine effektive Dicke (Dₙ) der strahlabgewandten Kondensatorplatte (34.n) kleiner ist als eine effektive Dicke (Dₙ) der strahlseitigen Kondensatorplatte (32.n).

12. Teilchenenergie-Messgerät (14) nach einem der vorstehenden Ansprüche, **gekennzeichnet durch** einen Kalibrierschein, in dem
(a) die Messunsicherheit für zumindest eine Teilchenenergie (E) und/oder
(b) Teilchenenergie (E) ein Breiten-Kalibrierfaktor (K), mittels dem aus dem Breitenmaß (B) die Teilchenenergie (E) berechenbar ist,
angegeben ist.

13. Teilchenenergie-Messgerät (14) nach einem der vorstehenden Ansprüche, **dadurch gekennzeichnet, dass** die Auswerteschaltung (58) ausgebildet ist zum Bewirken einer Entladung der Kondensatoren (30.n), wenn die Summenladung (*Q*_{Σ}) einen vorgegebenen Schwellenwert (*Q*_{Σ,max}) überschreitet.

14. Verfahren zum Ermitteln einer Strahlenergie eines Teilchenstrahls (26), mit den Schritten:
(a) Einfallen-Lassen des Teilchenstrahls (26) auf ein Teilchenenergie-Messgerät (14), das
(i) zumindest zwanzig Kondensatoren (30.n), die
- jeweils eine erste Kondensatorplatte (32) und
- eine zweite Kondensatorplatte (34) aufweisen und
- bezüglich einer Strahleinfallsrichtung (S) hintereinander angeordnet sind,
(ii) einen Multiplexer (46), der
- einen Multiplexer-Ausgang (48) und
- eine Mehrzahl an Multiplexer-Eingängen (50.n) hat, wobei jeder Multiplexer-Eingang (50) mit genau einem Kondensator verbindbar ist, und der
- eingerichtet ist zum Verbinden jeweils einer ersten Kondensatorplatte (32) des jeweiligen Kondensators mit dem Multiplexer-Ausgang (48),
(iii) einen Plattenladungsmesser (62), der
- einen Plattenladungsmesser-Eingang (64), der mit den zweiten Kondensatorplatten (34.n) verbunden ist, und
- einen Plattenladungsmesser-Ausgang (65) aufweist,
(b) sukzessives Messen der Ladungen (Qₙ) der Kondensatoren mittels des Plattenladungsmessers (62) durch sukzessives Verbinden der Kondensatoren mit dem Plattenladungsmesser (62) mittels des Multiplexers (46), sodass Ladungs-Daten erhalten werden, und
(c) Berechnen der Teilchenenergie (S) und/oder der Strahl-Ladung (Q₂₆) aus den Ladungs-Daten.

15. Verfahren nach Anspruch 14, **dadurch gekennzeichnet, dass**
(a) der Teilchenstrahl (26) aus Teilchen mit einer mittleren Teilchenenergie (E) besteht und
(b) die Kondensatorplatten gemeinsam eine solche Dicke (d) haben, dass eine Sammeleffizienz für Teilchen der mittleren Teilchenenergie (E) zumindest 96% beträgt.
